# EUROPEAN PATENT SPECIFICATION

(11) **EP 1 901 643 B1**
(45) Date of publication and mention of the grant of the patent: **03.02.2016**
(21) Application number: 05748201.0
(22) Date of filing: 03.06.2005
(51) Int. Cl.: A61B 5/00, A61N 1/08, A61N 1/372

(54) **MEDICAL APPARATUS AND SYSTEM**
MEDIZINISCHER APPARAT UND MEDIZINISCHES SYSTEM
APPAREIL ET SYSTEME MEDICAL

(43) Date of publication of application: 26.03.2008
(73) Proprietor: St. Jude Medical AB, 175 84 Järfälla (SE)
(72) Inventor: SNITTING, Tomas, 113 38 Stockholm (SE); SKOOG, Viktor, 165 70 Hässelby (SE); ABRAHAMSON, Hans, 113 38 Stockholm (SE)
(74) Representative: Sjölander, Henrik
(86) International application number: PCT/SE2005/000854
(87) International publication number: WO 2006/130060

(56) References cited:
- WO-A2-2006/039525
- US-A- 6 167 312
- US-A1- 2001 023 315
- US-A1- 2001 023 360
- US-A1- 2002 013 518
- US-A1- 2002 072 783
- US-A1- 2003 220 673
- US-A1- 2004 152 953

## Description

### Field of the invention

The present invention relates to the field of telemetry, and in particular to a medical apparatus for programming and/or monitoring an implantable medical device over a radio-based wireless network, and such a system.

### Background of the invention

Telemetry is a generic term for techniques for conveying measuring data from one point to another, usually by means of radio. In particular, within the medical field telemetry systems are generally used for enabling radio-frequency (RF) communication between an implantable medical device (IMD) such as a pacemaker, and an external monitoring device. The frequency spectrum used for wireless communications between implanted medical devices and external equipment is about 400 MHz, but for wireless medical telemetry services in general several frequency bands may be used. Within a medical telemetry system crucial physiologic data is transmitted, and it is critical to ensure that data is not lost or delayed. Medical telemetry is a low-power radio system, and although relatively short distances are usually employed within such systems, there may nevertheless arise a need for considering reception aspects. One such consideration is related to the fact that electromagnetic fields emitted in a room will give rise to standing wave patterns. The energy that a receiver will be able to receive is varying as a function of the position in the room. Using multiple antennas, resulting in so called spatial diversity, may minimize the effects of this.

US 2002/0013518 discloses a wireless patient monitor adapted to communicate with a medical telemetry network having a central station. The monitor includes sensor inputs for receiving vital signs data that is communicated to the central station over the telemetry network.

US 6,167,312 discloses such a device for use in communication with an implantable medical device. The device is provided with a spatial diversity antenna array including at least one antenna permanently and fixedly mounted to the housing of a monitor or programmer, and an additional antenna removably mounted to the housing.

### Summary of the invention

This known telemetry system, although suggesting the use of spatial diversity in order to facilitate the reception of signals from an implantable device and also the transmission of signals to the implanted device, still has several drawbacks regarding the signaling. For example, as mentioned above, the system comprises a removable antenna, but the use of it entails the physician having to move the antenna around until an acceptable reception is obtained. Therefore, should there arise a need to move a patient from one place to another, for example from an examination room to an X-ray examination room, the tedious reception/transmission optimization would have to be performed once more. Thus, the apparatus described requires the physician operating it to perform a kind of an antenna reception optimization, which is a time-consuming and also unreliable method. Further, the range of said removable antenna is limited, and dependent upon the length of a coiled cord by means of which the removable antenna is coupled to a transceiver within the programmer.

Furthermore, such a programmer is relatively expensive, and it would be desirable and convenient to be able to easily move the programmer, for example between different wards in a hospital, with retained communication quality, to thereby avoid having to buy several costly programmers.

It would thus be desirable to provide, in a telemetry- system, an improved two-way communication of signals between a monitoring device and an implantable medical device, both forming parts of a medical system for programming and/or monitoring the implantable medical device over a radio-based wireless network. In particular, it would be desirable to provide a reliable communication, which overcomes the above mentioned short-comings of the prior art.

It is an object of the present invention to provide a reliable radio communication within a telemetry system, which communication is easily and conveniently optimized, eliminating or at least reducing the risk of a communication failure between an implantable medical device and a monitoring device due to fading and/or a low signal strength.

It is a further object of the present invention to provide a medical apparatus and system, by means of which spatial diversity is achieved.

These objects, among others, are achieved by a medical apparatus, as claimed in claim 1, and by a system as claimed in claim 20.

In accordance with the present invention the above mentioned objects are achieved by a medical apparatus, comprising a monitoring device and at least two antenna devices, the medical apparatus enabling programming and/or monitoring of an implantable medical device over a radio- based wireless network. The at least two antenna devices comprised within the system are provided as separate, stand-alone units, i.e. not forming part of the programmer or monitoring device. Thereby it is possible to place the antenna devices in an optimal way, preferably at stationary locations, such as for example wall and/or ceiling mounted. The antennas may be placed in each room, or area of use, in which telemetry is utilized, for example an X-ray room, examination room or operating room, or even in the equipment utilized. Since, in accordance with the present invention, the distance between a patient and the programmer no longer is a consideration with regard to signal reception, the programmer may be easily moved from one place to another without thereby affecting the signal quality. The placement of the antennas may also be optimized in advance, in consideration of where in the respective rooms the patient usually is located.

The medical apparatus of the present invention further comprises a control unit provided for measuring a signal quality parameter of signals received from the implantable medical device by each of the antenna devices. Thereafter one of the antenna devices is selected for subsequent reception or transmission of signals from the implantable medical device depending on the measured signal quality parameter of the received signals. Spatial diversity is thereby ensured, and the antenna giving the best reception may be chosen and a reliable communication is provided.

In accordance with an embodiment of the present invention the signal quality parameter is any of RSSI, BER, or C/N ratio. A flexibility in how to chose a suitable antenna, i.e. in dependence on an optional parameter, is thereby provided. These parameters are commonly known.and often used in assessing signal quality, thus enabling the use of well established, easily obtainable algorithms.

In accordance with yet another embodiment of the present invention the control unit is utilized for measuring a signal quality parameter of signals received from the implantable medical device by each of the antenna devices at regular intervals, or continuously. It is thereby possible to rapidly detect a deteriorated signal quality and switch to an antenna having a better signal reception.

In accordance with yet another embodiment of the present invention the control unit is connected between the programmer or monitoring device and the antenna devices. In an alternative embodiment, the control unit is an integral part of the programmer. In yet a further embodiment, the control unit is provided as an integral part of either one of the antenna devices. This provides a modular structure, giving a great design flexibility, and enabling custom-made solutions.

In accordance with yet another embodiment of the present invention the communication links between the programmer or monitoring device and the control unit on one hand, and between the control unit and the antenna devices on the other hand, may be via wire, e.g. an USB connection, or wirelessly, e.g. via Bluetooth. This again adds to the design flexibility.

In accordance with yet another embodiment of the present invention each of the antenna devices comprises a radio transceiver unit. In another embodiment, only one transceiver unit is provided, preferably centrally located in a room, or other area of use. Utilizing several radio transceiver units provides an additional security, but if a less expensive solution is desired, a fewer number of radio transceiver units may be provided.

In accordance with still another embodiment of the present invention each of the antenna devices are fixedly mounted, for example in a ceiling or to a wall. Thereby the antenna devices may be more or less permanently placed at locations considered to be the best in view of reception/transmission from and to an implantable medical device. The reception/transmission may be optimized in advance, in dependence of an expected location in a room of the patient wearing the implantable medical device.

In accordance with still another embodiment of the present invention each of the antenna devices comprises a conductive radiating antenna element, and these conductive radiating antenna elements are adapted to emit and receive radio waves having essentially parallel polarization. Thereby spatial diversity is provided independently of polarization diversity.

In accordance with the present invention each of the antenna devices comprises at least one conductive radiating antenna element capable of emitting and receiving radio waves of orthogonal polarizations. If at least two conductive radiating antenna elements are provided in each antenna device they should be operatively provided adjacent to each other at a single location in space.

In accordance with still another embodiment of the invention the programmer or monitoring device is portable, and is in particular a hand held device. In accordance with the present invention, the antenna devices are not physically part of the programmer or monitoring device, which would, considering the frequencies in question, require a certain, non-portable size of the programmer in order to accommodate the fastening of several antennas to it. The size of the programmer may therefore be reduced in accordance with the invention. A user may thereby easily bring the programmer along, should such need arise. In another embodiment, the programmer or monitoring device is arranged on a movable rack such as a roller table or the like, whereby the present invention may be utilized also in connection with currently used programmer or monitoring devices, giving a solution easy to implement with existing programmers.

The present invention is also related to such a system, in accordance with which advantages corresponding to the above described are achieved.

### Brief description of the drawings

Figure 1 shows a schematic view of an embodiment of a medical apparatus in accordance with the present invention.
Figure 2 shows a schematic view of another embodiment of a medical apparatus in accordance with the present invention.
Figure 3 shows a system in accordance with the present invention.

### Detailed description of preferred embodiments

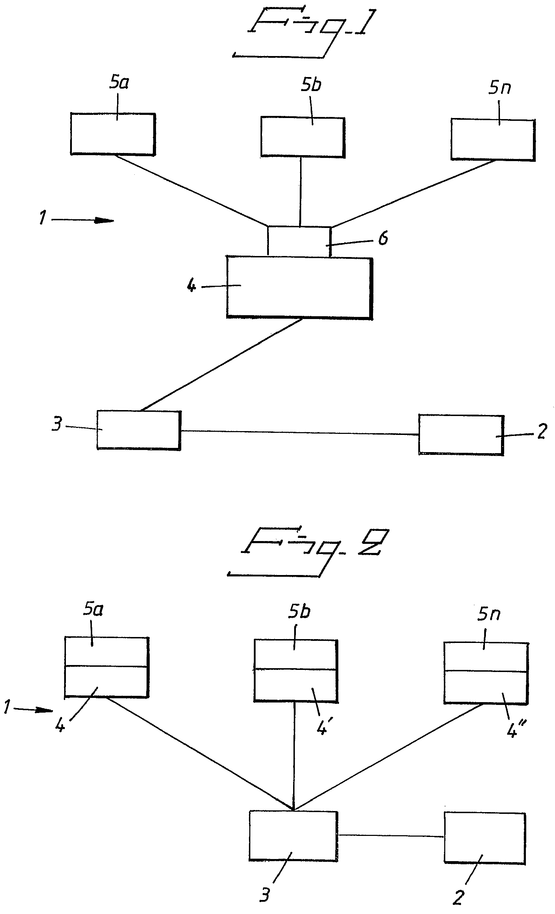

In the following description the same reference numerals will be used for equivalent or similar elements throughout the drawings. With reference first to figure 1, a schematic layout depicting an exemplary medical apparatus in accordance with the invention is shown. A medical apparatus 1 for programming and/or monitoring a patient related device (not shown), for example an implantable medical device, over a radio-based wireless network comprises a programmer or monitoring device 2, hereinafter referred to as a programmer 2. The programmer 2 is provided with input and/or output means for transmitting programming instructions to an implantable medical device, and/or for outputting monitoring information patient related data, for example for display on a screen, thereby enabling a physician to easily see such patient related data received from the implantable medical device.

A control unit 3, for example a microcontroller, is connected to the programmer 2, via wired standards, for example via USB (Universal Serial Bus), or via some wireless protocols. Bluetooth is such an exemplary, preferred wireless protocol, being an open-standard protocol. Using an open-standard protocol allows interoperability among devices from different manufacturers, which may be very advantageous in some cases. For example, utilizing Bluetooth standard for communication between the programmer 2 and antenna devices may permit the use of programmers from different producers, without also necessitating antenna device changes, which is particularly advantageous if the antenna devices are wall mounted or in some other manner more permanently mounted. In the embodiment shown in figure 1, the control unit 3 and the programmer 2 are shown as separate parts, but it is possible to, in an alternative embodiment, make the control unit 3 an integrated part of the programmer 2.

The control unit 3 is connected to at least one radio frequency circuitry unit 4, hereinafter called transceiver unit, via a digital link such as SPI (Serial Peripheral Interface), USB, Bluetooth or the like. The control unit 3 controls the one or more transceiver units 4. The transceiver unit 4 comprises conventional radio frequency circuitry, such as, for example, a duplexer, connected to a transmitter section and a receiver section, microcontroller, a wakeup transmitter, switches, low noise amplifiers (LNA), power amplifiers, AGC (Automatic Gain Control), power detectors and filters. The transceiver unit may also be an integral part of the contol unit 3.

The medical apparatus 1 further includes at least two antenna devices 5a, 5b, ..., 5n operatively provided at different locations, that is, they are provided as separate, stand alone units, i.e. not forming part of the programmer 2 as in the prior art. The programmer 2 is connected, via a control unit 3 and transceiver unit 4, to the antenna devices 5a, 5b, ..., 5n and is provided for transmitting signals to and receiving signals from an implantable medical device via either one of the antenna devices 5a, 5b, ..., 5n. The connection between the antenna devices 5a, 5b, ..., 5n and the programmer 2 is a wired connection, e.g. an USB connection, or a wireless connection, e.g. via Bluetooth. Thereby movements of the programmer 2 is enabled, while the antenna devices 5a, 5b, ..., 5n are kept still, for example being permanently mounted to a wall or the like. By means of the invention it is possible to place the antenna devices 5a, 5b, ... 5n in an optimal way, preferably at stationary locations, such as for example wall mounted. The antenna devices 5a, 5b, ... 5n may be placed in each room, or area of use, in which telemetry is utilized, for example an X-ray room, examination room or operating room, or even in the equipment utilized. Since, in accordance with the present invention, the distance between a patient and the programmer 2 is not a consideration with regard to signal reception anymore, the programmer 2 may be easily moved from one place to another without the signal quality being affected. The placement of the antenna devices 5a, 5b, ... 5n may also be optimized in advance, in consideration of where in a respective room the patient usually is located. For example, in an X-ray room the patient is most likely placed at a certain location known in advance, and the antenna devices 5a, 5b,..., 5n may be placed so as to optimize the reception/transmission in relation to this location. In the embodiment shown in figure 1 the antenna devices 5a, 5b, ..., 5n consist only of antenna elements, i.e. electrically conductive and radiating structures for transmitting and receiving radio frequency signals. These antenna elements can have any desired and appropriate shape, such as for example strip shape, cross shape or star shape. In the figure three such antenna elements are shown, but it is understood that any suitable number of antenna elements may be used. Further, each antenna device 5a, 5b,..., 5n may comprise means for enabling polarization diversity, for example by providing the antenna device 5a, ..., 5b, ..., 5n with conductive structures for emitting and/or receiving radiation of different polarizations. In this regard, reference is made to the pending International application, no. PCT/SE2004/000832, entitled "Medical transceiver device and method", having the same applicant as the present application.

The antenna devices 5a, 5b, ..., 5n are connected to the transceiver unit 4, the transceiver unit 4 being controlled by the control unit 3. A switch device 6, switchable between using one or more of the different antenna devices 5a, 5b,..., 5n is also included. When utilizing spatial diversity, advantage is taken of the different paths of a wave propagation in a reflective environment, and the antenna device 5a, 5b, ..., 5n giving the best reception at any time may be utilized. In accordance with the invention thus, the antenna device 5a, 5b,..., 5n giving the best communication link, as determined in a suitable way, is chosen for communication between the programmer 2 and an implantable medical device. The control unit 3 comprises circuitry for measuring characteristics of the radio frequency signals as received by the antenna devices 5a, 5b,..., 5n. Depending on a suitable signal quality indicator one of the antenna devices 5a, 5b, ..., 5n is chosen for the subsequent communication. The signal quality indicator or parameter may for example be one of: signal strength, bit error rate (BER), carrier-to-noise (C/N) ratio, carrier-to-interference (C/I) ratio or received signal strength indicators (RSSI). In an alternative embodiment, requiring more signal processing, the signals from two or more of the different antenna devices 5a, 5b,..., 5n are combined, i.e. the different paths are put in phase and then added. It is possible to perform regular polling of all antenna devices 5a, 5b,..., 5n or transceiver units 4 in order to keep track of the signal quality at different places in the room. In an alternative embodiment, the control unit is set on continuous listening of the antenna devices 5a, 5b,..., 5n or transceiver units 4.

Alternatively, the medical apparatus 1, and in particular the control unit 3 thereof, receives from an implantable medical device a measure of a signal quality parameter of signals as received by the implantable medical device, wherein the signals received by the implantable medical device are signals as transmitted from the medical apparatus to the implantable medical device after having been distorted by a transmission medium, i.e. the air interface between the respective antenna devices. The signal strength and the phase of the signals thereafter transmitted may be altered in dependence on the signal quality parameter of the signals as received by the implantable medical device.

In figure 1 a single transceiver unit 4 is shown, but in an alternative embodiment, shown in figure 2, several transceiver units 4, 4', 4" could be used. In fact, each antenna device 5a, 5b,..., 5n could comprise one or more antenna elements and one transceiver unit 4, 4', 4". The antenna devices 5a, 5b,..., 5n and their respective transceiver units 4, 4', 4" could form an integrated unit, as shown in figure 2, or be separated units. Utilizing several transceiver units enables the use of an ad-hoc structure, i.e. the antenna devices 5a, 5b,...5n, comprising antenna elements and a transceiver unit, constitutes autonomous nodes, thereby providing increased robustness in the communication.

In the embodiment of figure 2, there is no need for a switch device 6, since each antenna device 5a, 5b,..., 5n comprises a transceiver unit 4. In other respects, the embodiment of figure 2 is similar to the embodiment in figure 1.

The number of antenna devices 5a, 5b, ..., 5n may be different in different rooms, in dependence of the particular need in a certain room. For example, an exercise room used for monitoring the heart of a patient when subject to an increased heart rate, may be provided with a larger number of antennas, thereby increasing the spatial diversity and enabling the patient to freely move around within the room without risking a communication failure due to fading. In a smaller room, in which the patient is not moving around, it may suffice to use a single antenna device 5a, 5b, ..., 5n.

In accordance with the invention, the placing of the antenna devices 5a, 5b, ..., 5n may be optimized with regard to, on the one hand, the most probable placement of the patient in a room. As was mentioned above, the most probable location of the patient in a room may be readily determined for example in an x-ray examination room, in which the patient presumably is monitored when being in situ for being x-rayed. The antennas may be mounted on the walls, the ceiling or even within equipment such as x-ray equipment or a hospital bed, or in a hospital room, such as a waiting room or an operating room. Thereby it is easy to optimize the communication between the patient-related device and the antennas of the medical apparatus in advance. In addition, when positioning the antenna devices 5a, 5b, ..., 5n one should also consider near-field interference, and in particular their mutual coupling. Mutual coupling is pronounced up to a few wavelengths, and requires the space between adjacent antennas to be no less than a half-wavelength, the distance thus depending on the frequency in question. The signal at antenna device locations spaced a few wavelengths apart are almost independent, so increasing the distance between antennas would be beneficial. In accordance with the state of the art, the antennas are mounted to the programmer, whereby the distance between the antennas is limited to the size of the programmer. In contrast to this, the programmer 2 in accordance with the invention may be made portable, and in particular hand-held. Since the antenna devices 5a, 5b, ..., 5n are not physically part of the casing containing the programmer 2, i.e. not in physical contact with the programmer 2, there are no restrictions being placed on the size of the programmer 2 for accommodating a plurality of antennas. Therefore the size of the programmer may be reduced considerably, and a user may easily bring the programmer 2 along if desired. In particular, the antenna devices 5a, 5b,..., 5n may be placed at locations such that the distance between them is larger than the largest external length of the programmer, and also such that the antenna devices 5a, 5b,..., 5n are separated at least two wavelengths apart in order to achieve appropriate spatial diversity. However, it is to be understood that the programmer 2 may, in an alternative embodiment, have a state-of-the art size and be arranged on a movable rack such as a roller table or the like.

In the prior art referred to in the introductory part of the description, the distance between the patient and the programmer is critical. In fact, as soon as the programmer, which includes antennas permanently mounted to it, is moved relative the patient the signal reception has to be assessed once more. In accordance with the invention, there is no longer a need for such tedious optimization.

Although the medical apparatus in accordance with the invention has been described above utilizing antenna devices separated from the programmer, it does not exclude the additional use of antennas mounted to the programmer.

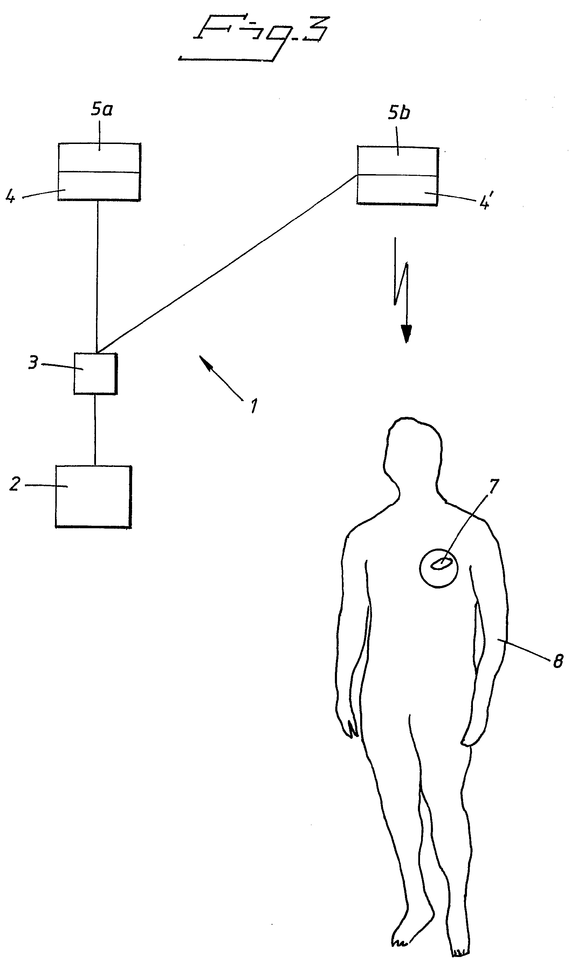

Figure 3 shows a system in accordance with the present invention. A medical apparatus 1 in accordance with the invention, comprising a programmer 2, a control unit 3, and antenna devices 5a, 5b,...,5n, with radio transceiver units 4, 4'...... are utilized for monitoring and/or transmitting programming instructions to a patient related device 7, here shown to be an implantable device, implanted into a patient 8. The implantable medical device 7 comprises a radio transceiver enabled for communication with the medical apparatus 1 of the present invention.

## Claims

1. A medical apparatus (1) for programming and/or monitoring an implantable medical device (7) over a radio-based wireless network comprising:
- a programmer or monitoring device (2) provided with input and/or output means for transmitting programming instructions to and/or receiving monitoring information from said implantable medical device (7);
- at least two antenna devices (5a, 5b, ..., 5n) operatively provided at different locations, wherein
- said programmer or monitoring device (2) is connected to said antenna devices (5a, 5b, ..., 5n) and is provided for transmitting signals to and receiving signals from said implantable medical device (7) via at least one of said antenna devices (5a, 5b, ..., 5n),
- each of said antenna devices (5a, 5b, ..., 5n) is provided as a physically separated unit wirelessly connected to said programmer or monitoring device (2), to thereby allow for movements of said programmer or monitoring device (2) relative said antenna devices (5a, 5b, ..., 5n),
- each of said antenna devices (5a, 5b, ..., 5n) comprises at least one conductive radiating antenna element capable of emitting and receiving radio waves of orthogonal polarizations, and
- a control unit (3) provided
- for measuring a signal quality parameter of signals received from said implantable medical device (7) by each of said antenna devices (5a, 5b, ..., 5n); and
- for selecting either one of said antenna devices (5a, 5b, ..., 5n) for subsequent reception of signals from said implantable medical device (7) depending on the measured signal quality parameter of said signals received by each of said antenna devices (5a, 5b, ..., 5n).

2. The medical apparatus of claim 1, wherein said control unit (3) is provided for selecting either one of said antenna devices (5a, 5b, ..., 5n) for transmission of signals to said implantable medical device (7) depending on the measured signal quality parameter of said signals received by each of said antenna devices (5a, 5b, ..., 5n).

3. The medical apparatus of claim 1 or 2, wherein said signals received by each of said antenna devices (5a, 5b, ..., 5n) comprise a measure of a signal quality parameter of signals as received from said medical apparatus (1) by said implantable medical device (7) after having been distorted by a transmission medium.

4. The medical apparatus of any of claims 1-3, wherein said signal quality parameter is any of RSSI, BER, or C/N ratio.

5. The medical apparatus of any of claims 1-4, wherein said control unit (3) is provided for measuring a signal quality parameter of signals received from said implantable medical device (7) by each of said antenna devices (5a, 5b, ..., 5n) regularly or continuously.

6. The medical apparatus of any of claims 1-5, wherein said control unit (3) is connected between said programmer or monitoring device (2) and said antenna devices (5a, 5b, ..., 5n).

7. The medical apparatus of any of claims 1-6, wherein said control unit (3) is provided as a separate unit connected to said programmer or monitoring device (2) via a first connection and connected to each of said antenna devices (5a, 5b, ..., 5n) via a second connection, wherein either both the first and the second connections are wireless, e.g. Bluetooth, or one of the first and second connections is wired, e.g. an USB connection, and the other connection is wireless, e.g. Bluetooth.

8. The medical apparatus of any of claims 1-6, wherein said control unit (3) is provided as an integral part of said programmer or monitoring device (2) and is wirelessly connected to each of said antenna devices (5a, 5b, ..., 5n), e.g. via Bluetooth.

9. The medical apparatus of any of claims 1-6, wherein said control unit (3) is provided as an integral part of either one of said antenna devices (5a, 5b, ..., 5n) and is connected to the other of said antenna devices (5a, 5b, ..., 5n) via wire, e.g. USB connections, or wirelessly, e.g. via Bluetooth and wirelessly connected to said programmer or monitoring device (2), e.g. via Bluetooth.

10. The medical apparatus of any of claims 1-9, wherein said control unit (3) is provided with radio-based circuitry for transmitting signals to and receiving signals from said implantable medical device (7).

11. The medical apparatus of any of claims 1-10, wherein each of said antenna devices (5a, 5b, ..., 5n) comprises at least one amplifier.

12. The medical apparatus of any of claims 1-10, wherein each of said antenna devices (5a, 5b, ..., 5n) comprises a radio transceiver unit (4).

13. The medical apparatus of any of claims 1-12, wherein each of said antenna devices (5a, 5b, ..., 5n) is configured to be fixedly mounted.

14. The medical apparatus of any of claims 1-13, wherein each of said antenna devices (5a, 5b, ..., 5n) is configured to be mounted in a ceiling or to a wall.

15. The medical apparatus of any of claims 1-14, wherein said control unit (3) is provided
- for measuring a signal quality parameter of signals received from said implantable medical device (7) by each of said conductive radiating antenna elements in each of said antenna devices (5a, 5b, ..., 5n); and
- for selecting a single one of said conductive radiating antenna elements in one of said antenna devices (5a, 5b, ..., 5n) for subsequent transmission of signals to and reception of signals from said implantable medical device (7) depending on the measured signal quality parameter of said signals received by each of said conductive radiating antenna elements in each of said antenna devices (5a, 5b, ..., 5n).

16. The medical apparatus of any of claims 1-15, wherein said programmer or monitoring device (2) is portable.

17. The medical apparatus of any of claims 1-16, wherein said programmer or monitoring device (2) is a hand-held device.

18. The medical apparatus of any of claims 1-17, wherein said input and/or output means comprise a display unit or screen and a key set or keyboard.

19. The medical apparatus on any of claims 1-18, wherein the distance between said at least two antenna devices (5a, 5b, ..., 5n) provided at different locations is larger than a maximum length of said programmer or monitoring device (2).

20. A medical communication system comprising an implantable medical device (7) and a medical apparatus (1) as claimed in any of claims 1-19, said implantable medical device (7) comprising a radio transmitter and/or receiver for communication with said medical apparatus (1).

## Patentansprüche

1. Medizinisches Gerät (1) zur Programmierung und/oder Überwachung eines implantierbaren Medizinprodukts (7) über ein drahtloses Funknetzwerk, bestehend aus:
- einer Programmier- oder Überwachungsvorrichtung (2), ausgestattet mit Eingangs- und/oder Ausgangsmitteln zur Übertragung von Programmierbefehlen und/oder zum Empfangen von Überwachungsinformationen vom implantierbaren Medizinprodukt (7);
- zumindest zwei Antennenvorrichtungen (5a, 5b, ..., 5n), die betriebsfähig an verschiedenen Stellen bereitgestellt werden, wobei
- die Programmier- oder Überwachungsvorrichtung (2) an die Antennenvorrichtungen (5a, 5b, ..., 5n) angeschlossen ist und dazu dient, über zumindest eine der Antennenvorrichtungen (5a, 5b, ..., 5n) Signale an das implantierbare Medizinprodukt (7) zu übertragen und von diesem zu empfangen,
- jede der Antennenvorrichtungen (5a, 5b, ..., 5n) als physikalisch gesonderte Einheit bereitgestellt, die kabellos an die Programmier- oder Überwachungsvorrichtung (2) angeschlossen ist, um dadurch Bewegungen der Programmier- oder Überwachungsvorrichtung (2) gegenüber den Antennenvorrichtungen (5a, 5b, ..., 5n) zu ermöglichen,
- jede der Antennenvorrichtungen (5a, 5b, ..., 5n) zumindest ein leitendes, strahlendes Antennenelement umfasst, das in der Lage ist, Radiowellen mit orthogonalen Polarisationen zu senden und zu empfangen, und
- eine Kontrolleinheit (3), die dazu bereitgestellt ist,
- einen Signalqualitätsparameter von Signalen zu messen, die eine jede der Antennenvorrichtungen (5a, 5b, ..., 5n) vom implantierbaren Medizinprodukt (7) empfängt; und
- eine der Antennenvorrichtungen (5a, 5b, ..., 5n) für den weiteren Empfang von Signalen vom implantierbaren Medizinprodukt (7) auf Basis des ermittelten Signalqualitätsparameters der Signale, die eine jede der Antennenvorrichtungen (5a, 5b, ..., 5n) empfangen hat, auszuwählen.

2. Medizinisches Gerät nach Anspruch 1, wobei die Kontrolleinheit (3) dazu vorgesehen ist, eine der Antennenvorrichtungen (5a, 5b, ..., 5n) in Abhängigkeit vom gemessenen Signalqualitätsparameter der Signale, die eine jede der Antennenvorrichtungen (5a, 5b, ..., 5n) empfangen hat, für die Übertragung von Signalen an das implantierbare Medizinprodukt (7) auszuwählen.

3. Medizinisches Gerät nach Anspruch 1 oder 2, wobei die Signale, die eine jede der Antennenvorrichtungen (5a, 5b, ..., 5n) empfangen hat, eine Messgröße eines Signalqualitätsparameters für Signale enthalten, wie sie das medizinische Gerät (1) vom implantierbaren Medizinprodukt (7) empfangen hat, nachdem diese durch ein Übertragungsmedium verzerrt wurden.

4. Medizinisches Gerät nach einem der Ansprüche 1 bis 3, wobei es sich beim Signalqualitätsparameter um RSSI, BER oder das C/N-Verhältnis handelt.

5. Medizinisches Gerät nach einem der Ansprüche 1 bis 4, wobei die Kontrolleinheit (3) dazu dient, einen Signalqualitätsparameter von Signalen zu ermitteln, die eine jede der Antennenvorrichtungen (5a, 5b, ..., 5n) vom implantierbaren Medizinprodukt (7) regelmäßig oder durchgehend empfängt.

6. Medizinisches Gerät nach einem der Ansprüche 1 bis 5, wobei die Kontrolleinheit (3) zwischen der Programmier- oder Überwachungsvorrichtung (2) und den Antennenvorrichtungen (5a, 5b, ..., 5n) angeschlossen ist.

7. Medizinisches Gerät nach einem der Ansprüche 1 bis 6, wobei die Kontrolleinheit (3) als getrennte Einheit bereitgestellt wird, die über eine erste Verbindung an die Programmier- oder Überwachungsvorrichtung (2) und über eine zweite Verbindung an eine jede der Antennenvorrichtungen (5a, 5b, ..., 5n) angeschlossen ist, wobei entweder beide von der ersten und der zweiten Verbindung kabellos, z.B. Bluetooth, sind oder entweder eine von der ersten und der zweiten Verbindung über Kabel, z.B. USB-Anschluss, erfolgt und die andere Verbindung kabellos, z.B. Bluetooth, ist.

8. Medizinisches Gerät nach einem der Ansprüche 1 bis 6, wobei die Kontrolleinheit (3) als fester Bestandteil der Programmier- oder Überwachungsvorrichtung (2) bereitgestellt wird und kabellos an eine jede der Antennenvorrichtungen (5a, 5b, ..., 5n) angeschlossen ist, z.B. über Bluetooth.

9. Medizinisches Gerät nach einem der Ansprüche 1 bis 6, wobei die Kontrolleinheit (3) als fester Bestandteil einer der Antennenvorrichtungen (5a, 5b, ..., 5n) bereitgestellt wird und über Kabel, z.B. USB-Anschlüsse, oder kabellos, z.B. über Bluetooth, an die andere der Antennenvorrichtungen (5a, 5b, ..., 5n), sowie kabellos, z.B. über Bluetooth, an die Programmier- oder Überwachungseinrichtung (2) angeschlossen ist.

10. Medizinisches Gerät nach einem der Ansprüche 1 bis 9, wobei die Kontrolleinheit (3) mit Funkverbindungen bereitgestellt wird, um Signale an das implantierbare Medizinprodukt (7) zu senden und Signale von diesem zu empfangen.

11. Medizinisches Gerät nach einem der Ansprüche 1 bis 10, wobei jede der Antennenvorrichtungen (5a, 5b, ..., 5n) mindestens einen Verstärker umfasst.

12. Medizinisches Gerät nach einem der Ansprüche 1 bis 10, wobei jede der Antennenvorrichtungen (5a, 5b, ..., 5n) eine Funk-Sende- und Empfangseinheit (4) umfasst.

13. Medizinisches Gerät nach einem der Ansprüche 1 bis 12, wobei jede der Antennenvorrichtungen (5a, 5b, ..., 5n) für die fixe Montage vorgesehen ist.

14. Medizinisches Gerät nach einem der Ansprüche 1 bis 13, wobei jede der Antennenvorrichtungen (5a, 5b, ..., 5n) für die Montage in einer Decke oder an eine Wand vorgesehen ist.

15. Medizinisches Gerät nach einem der Ansprüche 1 bis 14, wobei die Kontrolleinheit (3) dazu dient
- einen Signalqualitätsparameter von Signalen zu messen, die ein jedes der leitenden, strahlenden Antennenelemente in jeder der Antennenvorrichtungen (5a, 5b, ..., 5n) vom implantierbaren Medizinprodukt (7) empfängt; und
- ein einziges der leitenden, strahlenden Antennenelemente in einer der Antennenvorrichtungen (5a, 5b, ..., 5n) für die weitere Übertragung von Signalen an das implantierbare Medizinprodukt (7) und den weiteren Empfang von Signalen vom implantierbaren Medizinprodukt (7) in Abhängigkeit vom ermittelten Signalqualitätsparameter der Signale auszuwählen, die ein jedes der leitenden, strahlenden Antennenelemente in einer jeden der Antennenvorrichtungen (5a, 5b, ..., 5n) empfängt.

16. Medizinisches Gerät nach einem der Ansprüche 1 bis 15, wobei die Programmier- oder Überwachungsvorrichtung (2) tragbar ist.

17. Medizinisches Gerät nach einem der Ansprüche 1 bis 16, wobei die Programmier- oder Überwachungsvorrichtung (2) ein Handgerät ist.

18. Medizinisches Gerät nach einem der Ansprüche 1 bis 17, wobei das Eingangs- und/oder Ausgangsmittel eine Display-Einheit oder einen Bildschirm, sowie ein Tastenfeld oder eine Tastatur umfasst/umfassen.

19. Medizinisches Gerät nach einem der Ansprüche 1 bis 18, wobei der Abstand zwischen den mindestens zwei Antennenvorrichtungen (5a, 5b, ..., 5n), die an verschiedenen Orten bereitgestellt sind, größer ist als eine maximale Länge der Programmier- oder Überwachungsvorrichtung (2).

20. Medizinisches Kommunikationssystem, bestehend aus einem implantierbaren Medizinprodukt (7) und einem medizinischen Gerät (1) nach einem der Ansprüche 1 bis 19, wobei das implantierbare Medizinprodukt (7) einen Funksender und/oder Funkempfänger zur Kommunikation mit dem medizinischen Gerät (1) umfasst.

## Revendications

1. Appareil médical (1) permettant de programmer et/ou de surveiller un dispositif médical implantable (7) par un réseau sans fil à base d'ondes radio comprenant :
- un programmateur ou un dispositif de surveillance (2) équipé d'un système d'entrée et/ou de sortie pour transmettre les instructions de programmation audit dispositif médical implantable (7) et/ou pour recevoir les données de surveillance provenant dudit dispositif médical implantable (7) ;
- au moins deux dispositifs d'antenne (5a, 5b,...5n) situés de manière fonctionnelle à différents endroits, où
- ledit programmateur ou dispositif de surveillance (2) est connecté audits dispositifs d'antenne (5a, 5b,...5n) et est prévu pour transmettre les signaux audit dispositif médical implantable (7) et pour recevoir les signaux provenant dudit dispositif médical implantable (7) par l'intermédiaire de l'un desdits dispositifs d'antenne (5a, 5b,...5n),
- chacun desdits dispositifs d'antenne (5a, 5b,...5n) se présente sous la forme d'une unité physiquement distincte connectée sans fil audit programmateur ou dispositif de surveillance (2), pour permettre ainsi les mouvements dudit programmateur ou dispositif de surveillance (2) par rapport auxdits dispositifs d'antenne (5a, 5b,...5n),
- chacun desdits dispositifs d'antenne (5a, 5b,...5n) comprend au moins un élément d'antenne de rayonnement conducteur capable d'émettre et de recevoir des ondes radio de polarisations orthogonales, et
- une unité de commande (3) permettant
- de mesurer un paramètre qualitatif pour les signaux provenant du dispositif médical implantable (7) reçus par chacun desdits dispositifs d'antenne (5a, 5b,...5n) ; et
- de sélectionner l'un desdits dispositifs d'antenne (5a, 5b,...5n) pour la réception consécutive de signaux provenant du dispositif médical implantable (7) en fonction du paramètre qualitatif mesuré pour lesdits signaux reçus par chacun desdits dispositifs d'antenne (5a, 5b,...5n).

2. Appareil médical (1) selon la revendication 1, ladite unité de commande (3) permettant de sélectionner l'un desdits dispositifs d'antenne (5a, 5b,...5n) pour la transmission de signaux audit dispositif médical implantable (7) en fonction du paramètre qualitatif mesuré pour lesdits signaux reçus par chacun desdits dispositifs d'antenne (5a, 5b,...5n).

3. Appareil médical selon la revendication 1 ou 2, lesdits signaux reçus par chacun desdits dispositifs d'antenne (5a, 5b,...5n) comprenant une mesure d'un paramètre qualitatif des signaux tels qu'ils proviennent dudit appareil médical (1) par ledit dispositif médical implantable (7) après avoir été déformés par un milieu de transmission.

4. Appareil médical selon l'une quelconque des revendications 1 à 3, ledit paramètre qualitatif étant l'une quelconque des mesures RSSI, BER ou rapport C/N.

5. Appareil médical selon l'une quelconque des revendications 1 à 4, ladite unité de commande (3) permettant de mesurer un paramètre qualitatif pour les signaux provenant dudit dispositif médical implantable (7) reçus par chacun desdits dispositifs d'antenne (5a, 5b,...5n) de manière régulière ou en continu.

6. Appareil médical selon l'une quelconque des revendications 1 à 5, ladite unité de commande (3) étant connectée entre ledit programmateur ou dispositif de surveillance (2) et lesdits dispositifs d'antenne (5a, 5b,...5n).

7. Appareil médical selon l'une quelconque des revendications 1 à 6, ladite unité de commande (3) étant prévue en tant qu'unité distincte connectée audit programmateur ou dispositif de surveillance (2) par l'intermédiaire d'une première connexion et connectée à chacun desdits dispositifs d'antenne (5a, 5b,...5n) par l'intermédiaire d'une seconde connexion, la première et la seconde connexion étant des connexions sans fil, par exemple une connexion Bluetooth, ou l'une des deux connexions étant une connexion filaire, par exemple une connexion USB, et l'autre étant une connexion sans fil, par exemple une connexion Bluetooth.

8. Appareil médical selon l'une quelconque des revendications 1 à 6, ladite unité de commande (3) étant prévue en tant que partie intégrante dudit programmateur ou dispositif de surveillance (2) et étant connectée sans fil à chacun desdits dispositifs d'antenne (5a, 5b,...5n), par exemple par une connexion Bluetooth.

9. Appareil médical selon l'une quelconque des revendications 1 à 6, ladite unité de commande (3) étant prévue en tant que partie intégrante de l'un desdits dispositifs d'antenne (5a, 5b,...5n), et étant connectée à l'autre desdits dispositifs d'antenne (5a, 5b,...5n) par une connexion filaire, par exemple une connexion USB, ou une connexion sans fil, par exemple une connexion Bluetooth et étant connectée par une connexion sans fil audit programmateur ou dispositif de surveillance (2), par exemple par une connexion Bluetooth.

10. Appareil médical selon l'une quelconque des revendications 1 à 9, ladite unité de commande (3) étant équipée d'un circuit à base d'ondes radio pour transmettre les signaux vers ledit dispositif médical implantable (7) et recevoir les signaux provenant dudit dispositif médical implantable (7).

11. Appareil médical selon l'une quelconque des revendications 1 à 10, chacun desdits dispositifs d'antenne (5a, 5b,...5n) comprenant au moins un amplificateur.

12. Appareil médical selon l'une quelconque des revendications 1 à 10, chacun desdits dispositifs d'antenne (5a, 5b,...5n) comprenant une unité émettrice-réceptrice d'ondes radio (4).

13. Appareil médical selon l'une quelconque des revendications 1 à 12, chacun desdits dispositifs d'antenne (5a, 5b,...5n) étant conçu pour être monté de manière fixe.

14. Appareil médical selon l'une quelconque des revendications 1 à 13, chacun desdits dispositifs d'antenne (5a, 5b,...5n) étant conçu pour être monté à un plafond ou à un mur.

15. Appareil médical selon l'une quelconque des revendications 1 à 14, ladite unité de commande (3) permettant
- de mesurer un paramètre qualitatif des signaux provenant du dispositif médical implantable (7) reçus par chacun desdits éléments d'antenne de rayonnement conducteur dans chacun desdits dispositifs d'antenne (5a, 5b,...5n) ; et
- de sélectionner l'un desdits éléments d'antenne de rayonnement conducteur dans l'un desdits dispositifs d'antenne (5a, 5b,...5n) pour la transmission consécutive des signaux vers ledit dispositif médical implantable (7) et la réception des signaux provenant dudit dispositif médical implantable (7) en fonction du paramètre qualitatif mesuré pour lesdits signaux reçus par chacun desdits éléments d'antenne de rayonnement conducteur dans chacun desdits dispositifs d'antenne (5a, 5b,...5n).

16. Appareil médical selon l'une quelconque des revendications 1 à 15, ledit programmateur ou dispositif de surveillance (2) étant un dispositif portable.

17. Appareil médical selon l'une quelconque des revendications 1 à 16, ledit programmateur ou dispositif de surveillance (2) étant un dispositif portatif.

18. Appareil médical selon l'une quelconque des revendications 1 à 17, ledit système d'entrée et/ou de sortie comprenant une unité d'affichage ou un écran et une machine à enregistrement indirect ou un clavier.

19. Appareil médical selon l'une quelconque des revendications 1 à 18, la distance entre les deux dispositifs d'antenne (5a, 5b,...5n) prévue en différents points étant supérieure à la longueur maximale dudit programmateur ou dispositif de surveillance (2).

20. Système de communication médicale comprenant un dispositif médical implantable (7) et un appareil médical (1) selon l'une quelconque des revendications 1 à 19, ledit dispositif médical implantable (7) comprenant un transmetteur et/ou un récepteur à ondes radio pour communiquer avec ledit appareil médical (1).
